# EUROPEAN PATENT SPECIFICATION

(11) **EP 0 853 956 B1**
(45) Date of publication and mention of the grant of the patent: **07.07.2004**
(21) Application number: 98200582.9
(22) Date of filing: 14.04.1993
(51) Int. Cl.: A61M 29/02, A61M 25/00

(54) **Low profile dilation catheter**
Dilatationskatheter mit geringem Querschnitt
Catheter de dilatation extra-plat

(30) Priority: 20.04.1992 US 870820
(43) Date of publication of application: 22.07.1998
(62) Divisional of application: 93910621.7
(73) Proprietor: ADVANCED CARDIOVASCULAR SYSTEMS, INC., Santa Clara, California 95052-8167 (US)
(72) Inventor: Sirhan, Motasim M., Sunnyvale, CA 94087 (US); Thornton, Troy L., Foster City, CA 94404 (US); Campbell, Patrick K., Cupertino, California 95014 (US)
(74) Representative: Jenkins, Peter David

(56) References cited:
- US-A- 4 877 031
- US-A- 4 892 519
- US-A- 5 071 406

## Description

This invention generally relates to intravascular catheters, such as balloon dilatation catheters used in percutaneous transluminal coronary angioplasty (PTCA).

In classic PTCA procedures, a guiding catheter having a preshaped distal tip is percutaneously introduced into the cardiovascular system of a patient and advanced therein until the preshaped distal tip of the guiding catheter is disposed within the aorta adjacent the ostium of the desired coronary artery. The guiding catheter is twisted or torqued from the proximal end to turn the distal tip of the guiding catheter so that it can be guided into the coronary ostium. A guidewire and a balloon dilatation catheter are introduced into and advanced through the guiding catheter to the distal tip thereof, with the guidewire slidably disposed within an inner lumen of the dilatation catheter. The guidewire is first advanced out the distal tip of the guiding catheter, which is seated in the ostium of the patient's coronary artery, until the distal end of the guidewire crosses the lesion to be dilated. The dilatation catheter is then advanced out of the distal tip of the guiding catheter, over the previously advanced guidewire, until the balloon on the distal extremity of the dilatation catheter is properly positioned across the lesion. Once properly positioned, the balloon is inflated to a predetermined size with radiopaque liquid at relatively high pressures (e.g., generally 405-1216 kN/m² [4-12 atmospheres]) to dilate the stenosed region of the diseased artery. The balloon is then deflated so that the dilatation catheter can be removed from the dilated stenosis and blood flow will resume therethrough.

Further details of guiding catheters, dilatation catheters, guidewires, and other devices for angioplasty procedures can be found in U.S. Patent 4.323.071 (Simpson-Robert: U.S. Patent 4,439,185 (Lundquist); U.S. Patent 4,468,224 (Enzmann *et al*.); U.S. Patent 4,516,972 (Samson); U.S. Patent 4,438,622 (Samson *et al*.); U.S. Patent 4,554,929 (Samson *et al*.); U.S. Patent 4,582,185 (Samson); U.S. Patent 4,616,652 (Simpson); U.S. Patent 4,638,805 (Powell); U.S. Patent 4,748,986 (Morrison *et al*.); U.S. Patent 4,898,577 (Badger *et al*.); and U.S.Patent 4,827,943 (Taylor *et al*.).

The assignee of the present invention, Advanced Cardiovascular Systems, Inc.. markets an improved dilatation catheter under the trademark ACS RX® which is described and claimed in U.S. Patent 5,040,548 (Yock), U.S. Patent 5,061,273 (Yock), and U.S. Patent 4,748,982 (Horzewski *et al*.). This dilatation catheter has a short guidewire receiving sleeve or inner lumen extending through a distal portion of the catheter. The sleeve or inner lumen extends proximally a distance of at least about 10 cm and usually not more than about 50 cm from a first guidewire port in the distal end of the catheter to a second guidewire port in the catheter spaced proximally from the inflatable member of the catheter. Preferably, a slit is provided in the wall of the catheter body which extends distally from the second guidewire port, preferably to a location proximal to the proximal end of the inflatable balloon. The structure of the catheter allows for the rapid exchange of the catheter without the need for an exchange wire or adding a guidewire extension to the proximal end of the guidewire. The design of this catheter has widely praised by the medical profession and has been met with much success in the market place because of the advantages of its unique design.

Another modification, which was introduced into the market place by the assignee of the present application provides a plurality of perfusion ports in the wall forming at least part of the catheter body proximal to the balloon. These perfusion ports are in fluid communication with an inner lumen extending to the distal end of the catheter body. A plurality of perfusion ports are preferably provided in the catheter body distal to the balloon which are also in fluid communication with the inner lumen extending to the distal end of the catheter body. When the balloon on the distal extremity of the dilatation catheter is inflated to dilate a stenosis, oxygenated blood in the artery or the aorta or both, depending upon the location of the dilatation catheter within the coronary anatomy, is forced to pass through the proximal perfusion ports, through the inner lumen of the catheter body and out the distal perfusion ports. This provides oxygenated blood downstream from the inflated balloon to thereby prevent or minimize ischemic conditions in tissue distal to the catheter. As is appreciated by those skilled in the art, tissue distal to a stenosis is frequently already in jeopardy due to ischemic conditions which may exist. As a result, care must be exercised in sizing the perfusion ports and the inner lumen to ensure that there is adequate flow of oxygenated blood to tissue distal to the catheter to eliminate or minimize ischemic conditions.

US-A-4892519 discloses a catheter as described above. The catheter comprises inner and outer tubular members which define two lumens for the passage of blood and inflating fluid, respectively. At least a portion of the inner periphery of the outer tubular member is secured to the exterior of the inner tubular member.

US-A-5071406 discloses a catheter having the features of the pre-characterising portion of claim 1.

A major and continual thrust of development work in the field of intravascular catheters, particularly angioplasty catheters, has been to reduce the profile, i.e. transverse dimensions, of such catheters and to improve the flexibility thereof without detrimentally affecting the pushability, particularly in the distal portion, of such catheters. A reduction in profile with no loss in pushability allows an intravascular catheter to be advanced much further into a patient's vasculature and to cross much tighter lesions in the case of angioplasty catheters.

Despite many advances in this field, the need for lower profile intravascular catheters having greater flexibility with little or no loss in pushability remains. The present invention satisfies this need.

The present invention provide an elongated catheter for performing an angioplasty procedure comprising: (a) an elongated catheter shaft which has a distal shaft section with an inner tubular member having a distal end, a port in the distal end and a guidewire receiving lumen extending in said inner tubular member to said distal port; an outer tubular member having a portion of its inner periphery along a length of the distal shaft section secured to the exterior of the inner tubular member; and (b) a dilatation balloon on the distal shaft section distal to said length and having an interior chamber therein: characterised in that the outer tubular member is disposed about the inner tubular member and the inner periphery of the outer tubular member has a plurality of unsecured portions which are not secured to the exterior of the inner tubular member resulting in a plurality of inner lumens longitudinally extending along and between said inner and outer tubular members.

Preferably, at least one of said inner lumens is an inflation lumen in fluid communication with the interior chamber of said dilatation balloon.

Preferably, the plurality of inner lumens are parallel to and off-set from the guidewire lumen defined by the inner tubular member.

Preferably, the distal shaft section has along the length thereof one transverse dimension in a first direction substantially larger than a second transverse dimension in a second direction perpendicular to the first direction.

More preferably, the dimension in the first transverse direction is at least 15% greater than the dimension in the second transverse direction perpendicular to the first direction.

Preferably, the guidewire receiving lumen and the inner lumens are in a stacked configuration within the distal shaft section.

Preferably, the catheter shaft has a second port which is located closer to the dilatation balloon than the proximal end of the catheter shaft and which is in communication with the guidewire receiving lumen.

Preferably, at least two of the plurality of inner lumens extend to the proximal end of the catheter.

Preferably, from 5% to 90% of the inner periphery of the outer tubular member is secured to the exterior of the inner tubular member.

More preferably, from 30% to 80% of the inner periphery of the outer tubular member is secured to the exterior of the inner tubular member.

The length of the secured portion should not be less than 5 mm and is preferably about 10 to 40 cm. While in a presently preferred embodiment described herein the secured portion of the outer tubular member is limited to the distal section of the catheter, the secured section may extend along essentially the entire length of the catheter.

By securing a substantial part of the outer tubular member to the exterior of the inner member along a length thereof to form a fused design in at least the distal portion of the catheter, the profile of the catheter body in at least one transverse dimension in that area is reduced substantially to thereby provide improved flexibility in at least one direction. Moreover, the secured portions of the inner member and the outer tubular members support one another to provide improvements in the pushability of the catheter. By virtue of the transverse dimension in one direction being substantially larger than the transverse dimension in a second direction at right angles to the first direction the cross-sectional shape of the catheter provides substantial improvements in flexibility, trackability and pushability. The maximum dimension of the catheter shaft should be preferably at least 15% greater than the minimum dimension, more preferably at least 25% greater.

Maximum cross-sectional dimensions of the small diameter section of the outer tubular member for coronary dilatation catheters are on the order of about 0.02 to about 0.06 in (0.51 - 1.5 mm). For peripheral arteries this dimension may be larger.

With catheter designs of the invention having the capability for rapid exchangeability, as described in the previously described Horzewski *et al*. patent, there is a tendency for the peel-away slit to open up or expand upon the introduction of the inflation fluid at high pressures into the inflation lumen defined by the unsecured portion of the outer tubular member. To avoid this problem the inflation lumen defined by the unsecured part of the distal section may be provided with a support tube so that there is essentially no expansion of the inflation lumen and therefore there is little tendency for the peel-away slit to open up or expand.

The improvements of the invention are applicable to a wide range of intravascular catheters and particularly to essentially all types of dilatation catheters with inflatable or expandable members, such as those described in the patents identified herein. These and other advantages of the invention will become more apparent from the following detailed description of the invention when taken in conjunction with accompanying exemplary drawings.

The invention will now be described, by way of example only, with reference to the accompanying drawings in which:-
Fig. 1 is an elevational view, partially in section, of a balloon dilatation catheter not in accordance with the invention.
Fig. 2 is a transverse cross-sectional view of the catheter shown in Fig. 1 taken along the lines 2-2.
Fig. 3 is a transverse cross-sectional view of the catheter shown in Fig. 1 taken along the lines 3-3.
Fig. 4 is a transverse cross-sectional view of the catheter shown in Fig. 1 taken along the lines 4-4.
Fig. 5 is a transverse cross-sectional view of the catheter shown in Fig. 1 taken along the lines 5-5.
Fig. 6 is an elevational view, partially in section, of another dilatation catheter not in accordance with the invention.
Fig. 7 is a transverse cross-sectional view of the catheter shown in Fig. 6 taken along the lines 7-7.
Fig. 8 is a transverse cross sectional view of the catheter shown in Fig. 6 taken along the lines 8-8.
Fig. 9 is a transverse cross-sectional view of the catheter shown in Fig. 6 taken along the lines 9-9.
Fig. 10 is a transverse cross-sectional view of the catheter shown in Fig. 6 taken along the lines 10-10.
Fig. 11 is an elevational view, partially in section, of another dilatation catheter not in accordance with the invention.
Fig. 12 is a transverse cross-sectional view of the catheter shown in Fig. 11 taken along the lines 12-12.
Fig. 13 is a transverse cross-sectional view of the catheter shown in Fig. 11 taken along the lines 13-13.
Fig. 14 is a transverse cross-sectional view of the catheter shown in Fig. 11 taken along the lines 14-14.
Fig. 15 is a transverse cross-sectional view of the catheter shown in Fig. 11 taken along the lines 15-15.
Fig. 16 is an elevational view, partly in section, of a catheter not in accordance with the present invention. The catheter is a modification of the catheter shown in Figs. 6-10.
Fig. 17 is a transverse cross-sectional view of the catheter shown in Fig. 16, taken along the lines 17:17.
Fig. 18 is a transverse cross-sectional view of a modification of the catheter shown in Fig. 1 in accordance with an embodiment of the present invention wherein two inner lumens are provided in the distal section between the inner and outer tubular members.

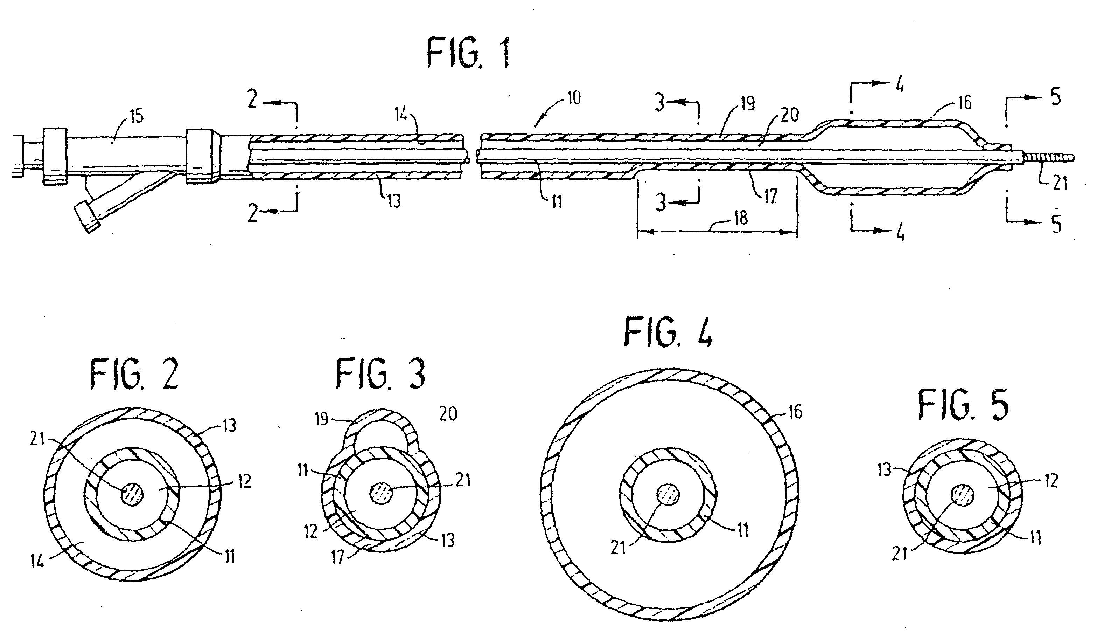

Figs. 1-5 schematically illustrate an over-the-wire dilatation catheter not in accordance with the invention. The catheter includes an elongated catheter shaft 10 which has an inner tubular member 11 with an inner lumen 12, an outer tubular member 13 disposed about the inner tubular member and defining therebetween an annular inner lumen 14 which extends through the proximal portion of the catheter shaft. An adapter 15 is secured to the proximal end of the elongated catheter body 10. A relatively inelastic, inflatable balloon 16 is formed as part of the outer tubular member 13 with the distal end of the balloon secured to the distal end of the inner tubular member 11. The balloon 16 may be formed from the same tubing as the outer tubular member 13 as shown in Fig. 1 or it may be made separately and secured to the distal end of a tubular shaft which forms part of the outer tubular member.

The outer tubular member 13 generally has a distal section 17 which is secured to the exterior of the inner tubular member 11 as best shown in Figs. 1 and 3 along a length 18 to provide smaller transverse dimensions in at least one direction. The distal section 17 is secured to the exterior of the inner tubular member 11 with a significant portion of the periphery outer member along the length 18, typically about 50% to about 80%, being secured to the inner member. The unsecured portion 19 of the distal section 17 along the length 18 forms an inflation lumen 20 which is in fluid communication with the interior of the balloon 16 and the annular lumen 14.

The use of the dilatation catheter shown in Figs. 1-5 may generally follow conventional PTCA practices with over-the-wire dilatation catheters. In the conventional practices a guidewire 21 is backloaded into the inner lumen 12 of the inner tubular member 11 of the catheter body 10 and both the guidewire and the catheter are advanced together through a guiding catheter (not shown) which has been previously disposed within the patient's arterial system, with the distal end of the guiding catheter seated within the ostium of the desired coronary artery. The guidewire 21 is first advanced out the distal end of the guiding catheter into the patient's coronary anatomy until it extends beyond the lesion to be dilated, and then the dilatation catheter is advanced over the guidewire which is being held in its position, until the balloon 16 on the dilatation catheter is properly disposed within the stenotic region so that the lesion is dilated upon the inflation of the balloon. After dilatation, the balloon 16 is deflated and the catheter and the guidewire 21 are withdrawn from the patient. If further treatment or diagnosis is to be conducted, the guidewire 21 can be replaced with an exchange wire before removing the dilatation catheter so that the first catheter can be removed and another advanced into the desired location or an extension wire can be attached to the proximal end of the guidewire in place to perform essentially the same function. For further details see the discussion of exchange wires and extension wires in U.S. Patent 4,827,941 (Taylor *et al*.).

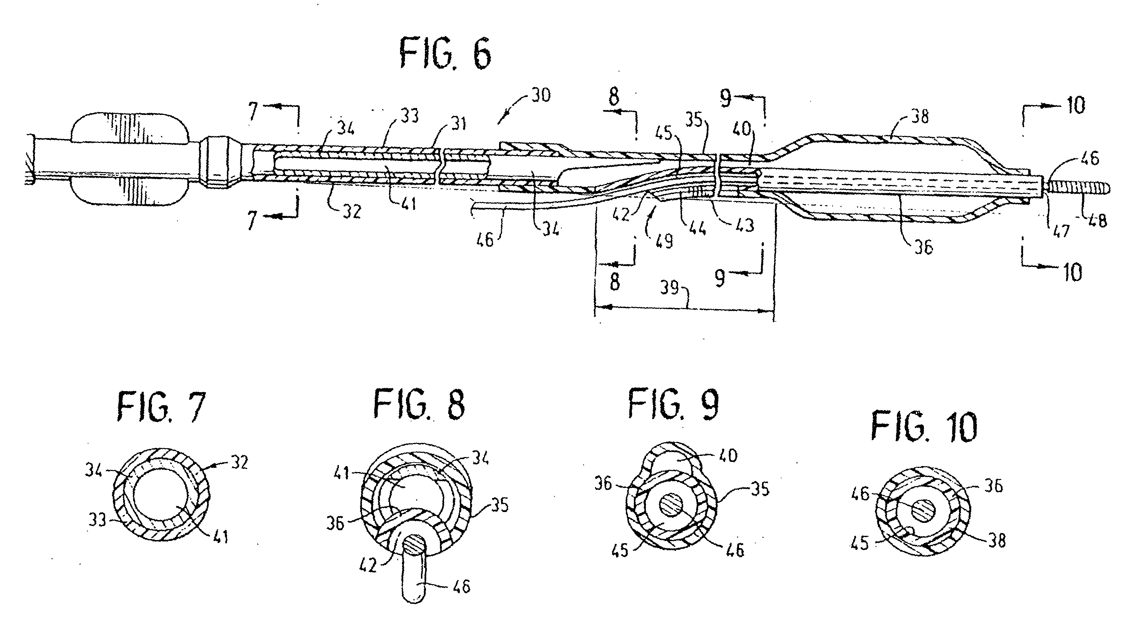

Figs. 6-10 schematically illustrate another dilatation catheter not in accordance with the invention which is quite similar in its most distal structure to the embodiment shown in Figs. 1-5. In the catheter shown in Figs. 6-10, the catheter shaft 30 includes an outer tubular member 31 which has a two layered proximal portion 32 with an outer plastic tubular jacket or coating 33 which fits tightly, *e*.*g*. is shrunk fit, unto a tubular element 34 which may be formed of hypotubing. The outer tubular member 31 also includes a distal section 35 which is secured about the inner tubular member 36 as in the previous embodiment. The distal section 35 may be the proximal skirt of the balloon 38, as shown in the drawing, or it may be a separate tubular member. The relatively inelastic balloon 38 is secured by its distal end to the distal end of the inner tubular member 36 which extends through the interior thereof. A significant portion of the interior surface of the distal section 35 along the length 39 is secured to the exterior of the inner tubular member 36 in accordance with the requirements of the invention. The inner tubular member 36 of this embodiment is quite short compared to the inner tubular of the embodiment shown in Figs. 1-5. The unsecured portion of the distal section 35 forms an inflation lumen 40 which is in fluid communication with the lumen 41 in the proximal section 32 of the outer tubular member 31 and the interior of the balloon 38.

In the catheter shown in Figs. 6-10, the catheter body 30 is provided a guidewire port 42 which passes through the secured walls 43 and 44 of the inner tubular member 36 and the distal section 35 of the outer tubular member 31 respectively and which is in communication with a relatively short inner lumen 45 extending within the inner tubular member 36. Guidewire 46 extends through the inner lumen 45 and out the proximal port 42 and a distal guidewire port 47. A coil 48 is provided on the distal end of the guidewire 46.

The inner tubular element 34 onto which the outer plastic tubular element 33 is secured is preferably hypotubing and may be formed of conventional stainless steel or a NiTi alloy, particularly a NiTi alloy with superelastic properties.

The above-described catheter construction with a relatively short inner lumen 45, adapted to slidably receive the guidewire 46, eliminates the need for using an exchange wire or a guidewire extension. A peel-away slit 49 is preferably provided in the secured walls 43 and 44 of the inner and outer tubular members 36 and 31 which extend from the guidewire port 42 to a location proximal to the proximal end of the balloon 38. The peel-away slit 49 facilitates the removal of the catheter from the proximal end of the guidewire 46 when the catheter is to be replaced or exchanged for another catheter and it also eliminates the need for using a guidewire extension or an exchange wire as described in Horzewski *et al*.

A dual lumen type construction such as described in Horzewski *et al*. may also be used in the portion of the catheter proximal to the guidewire port 42.

There are at least two modes of inserting the dilatation catheter shown in Figs. 6-10 into the patient's coronary anatomy. The first method is for the most part the same as in the previously described catheter, namely, the guidewire 46 is preloaded into the short inner lumen 45 of the inner tubular member 36 of the catheter body 30 and both are advanced through a guiding catheter (not shown) previously disposed within the patient's arterial system with the distal end of the guiding catheter seated within the ostium of a coronary artery. The second mode, frequently called the "bare wire" technique, involves first advancing a guidewire 46 through and out the guiding catheter until it is positioned within the patient's coronary artery across the lesion to be dilated. The proximal end of the guidewire 46, which is outside the patient, is backloaded, *i*.*e*. inserted into the short inner lumen 45 of the inner tubular member 36 through the distal guidewire port 47 and advanced proximally therein until it exits the proximal guidewire port 42. The proximal end of the guidewire 46 is held in place and the catheter is advanced over the guidewire through the patient's vascular system until the dilatation balloon 38 on the catheter is positioned across the stenotic region so that the stenosis can be dilated upon the inflation of the balloon. After the dilatation of the lesion, the balloon 38 is deflated and the catheter may be removed from the patient's artery. If other treatments are necessary, the catheter is slidably removed over the guidewire 46, leaving the guidewire in place so that other catheters can be advanced over the in-place guidewire in a similar manner without the need for exchange wires or guidewire extensions.

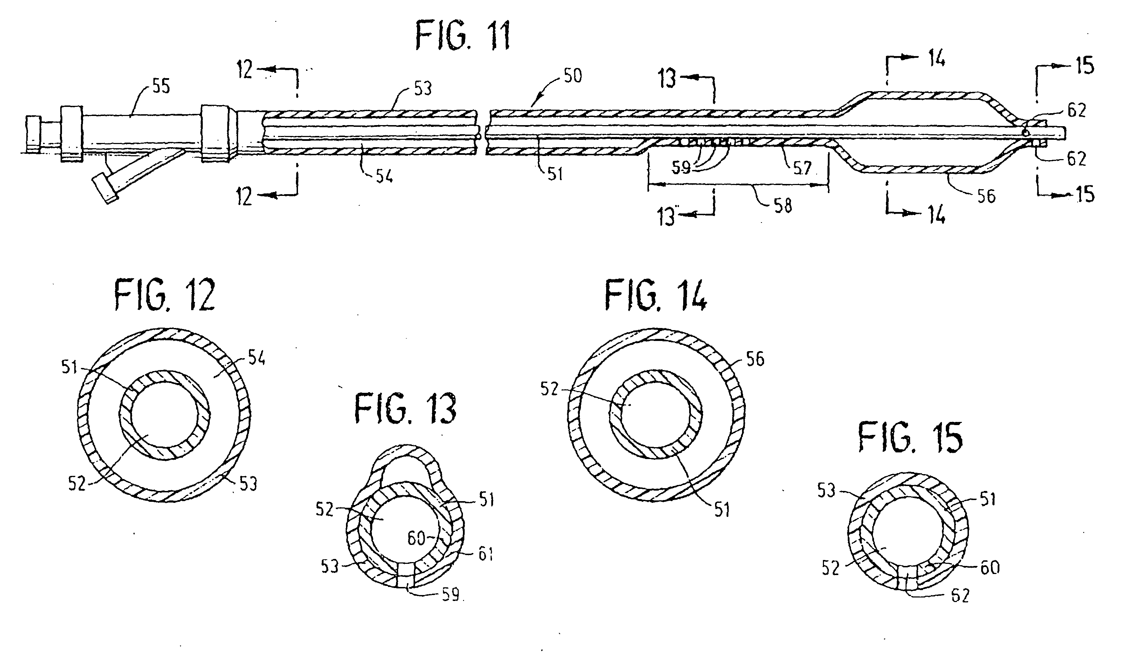

Figs. 11 through 15 illustrate yet another dilatation catheter not in accordance with the invention which provides for the perfusion of blood distal to the catheter during the dilatation of a stenotic lesion. The catheter includes the catheter shaft 50, an inner tubular member 51, with an inner lumen 52, an outer tubular member 53 which is disposed about the inner tubular member and which defines an annular lumen 54 located between the inner and outer tubular members in the proximal portion of the catheter shaft, an adapter 55 secured to the proximal ends of the inner and outer members, and a relatively inelastic balloon 56 which is secured by its distal end to the distal end of the inner tubular member 51. The outer tubular member 53 has a distal section 57, a length of 58 of which is secured to the exterior of the inner tubular member 51, as in the two catheters previously described. The above-described portion of this catheter has essentially the same structure as the catheters shown in Figs. 1-10. The distal section may be formed from the proximal skirt of the balloon 56 or may be formed from a separate tubular element with the proximal end of the balloon being secured to the distal end of the separate tubular element.

The dilatation catheter shown in Figs. 11-15 differs from the other catheters in that it has a plurality of perfusion ports 59 proximal to the balloon 56 which pass through the bonded walls 60 and 61 of the inner and outer tubular members 51 and 53 respectively and which are in fluid communication with the inner lumen 52 of the inner tubular member 51. Additionally, one or more perfusion ports 62 are provided distal to the balloon 56 through the wall 60 of the inner tubular member 51 and are in fluid communication with the inner lumen 52 extending therein. With this construction, when the balloon 56 is inflated, *e*.*g*. during an angioplasty procedure, within a patient's vasculature, oxygenated blood is forced to pass through the proximal perfusion ports 59, through the inner lumen 52 and then out the distal perfusion ports 62 to provide oxygenated blood distal to the catheter and thereby avoid the generation of ischemic conditions in tissue downstream thereof or the aggravation of existing ischemic conditions. The transverse dimensions of the inner tubular member 51 within the secured section are preferably larger than in the catheters previously discussed to allow for an increased flow of blood therethrough.

The use of the catheter shown in Figs. 11-15 is essentially the same as the catheter shown in Figs. 1-5. The only essential difference is that the balloon 56 can be inflated for significantly longer periods, e.g. typically about 20-30 minutes but possibly up to 5 hours or more, than the first described catheter because oxygenated blood is flowing to the tissue distal to the inflated balloon.

The dilatation catheter shown in Figs. 11-15 may be modified by providing a guidewire port at the proximal end of the section 58, proximal to the portion of the small diameter distal section 57 in which the proximal perfusion ports 59 are located, as shown in Figs 6-10. However, the guidewire port should preferably be spaced sufficiently far proximally from the portion of the bonded distal section 57 having the perfusion ports 59 so that the guidewire can be pulled proximally and remain within the inner lumen 52 while the balloon 56 is inflated during the dilatation but not interfere with the flow of blood through the perfusion ports 59 and 62 and the inner lumen 52. After the angioplasty procedure is completed, the guidewire can then be advanced distally through the inner lumen 52 and out the distal end thereof in order to maintain access to the lesion in case further treatment or diagnosis is necessary or desirable.

The use of the catheter with both perfusion ports and a proximal guidewire port as described above is essentially the same as the use of the dilatation catheter illustrated in Figs. 6-10, but with the additional advantage that long term dilatations are possible.

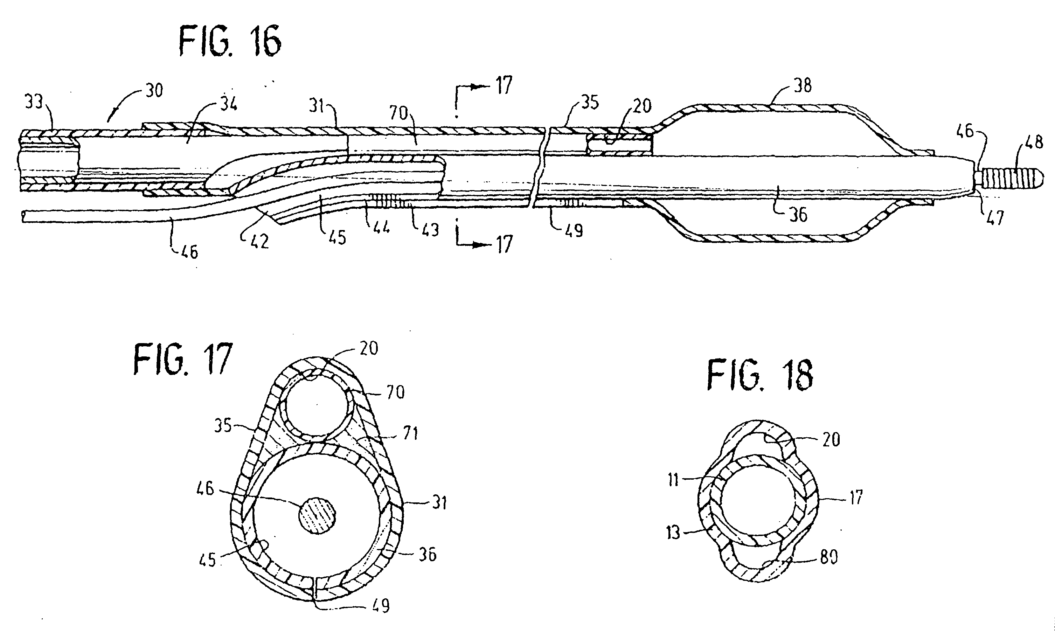

With those catheters which have a slit 49 in the secured section, such as shown in Figs 6-10, to facilitate the removal of the catheter from a guidewire, there is a tendency for the slit to open up when the pressure within the inflation lumen is raised to high levels to inflate the balloon 36 for purposes of dilatation. In these instances, the guidewire can extend through the expanded or opened slit 49 and be caught in the slit when it closes upon the deflation of the balloon so as to preclude independent movement of the guidewire and catheter. In the catheter illustrated in Figs. 16 and 17 the provision of the second inner tubular member (support tube) 70 within the inner lumen avoids this problem. The second inner tubular member, which forms the inflation lumen 40, prevents the expansion of the unsecured distal section 35 of the outer tubular member 31. Thus the expansion of the slit 49, upon the introduction of high pressure inflation fluid into the inflation lumen, is prevented. A filler 71 may be provided to eliminate any voids between the second inner tubular member 70 and the unsecured portion of the distal tubular section 35. Preferably, the second inner tubular member 70 is formed of polyimide but tubes formed of other polymers and metals such as superelastic NiTi alloys may be used. The rest of the catheter shown in Figs. 16 and 17 is essentially the same as shown in Figs. 6-10 and the corresponding parts are numbered the same.

The above-described catheters may be made by conventional techniques well known to those skilled in the art. Many suitable techniques are described in the references identified herein. The distal section may be formed by heat shrinking the portion of the outer tubular member which forms the distal section onto the underlying inner member. A mandrel (not shown) is disposed in the space between the inner and outer tubular members so that, upon the heat shrinking of the outer tubular member, an inflation lumen is formed through the distal section which is in fluid communication with the lumen in the proximal portion of the catheter shaft and the interior of the balloon. The heat shrinking secures the distal section of the outer tubular member to the inner tubular member. Other means of bonding such as heat bonding or adhesives may be used. A mandrel may also be inserted into the inner lumen of the inner tubular member to support the latter during the heat shrinking of the outer tubular member thereon. Alternate methods may be employed to make the distal section. For example, the distal section of the outer tubular member may be preformed and then be adhesively bonded to the exterior of the inner tubular member 36.

As shown in Fig. 18 in accordance with the present invention other lumens 80, similar to the inflation lumen may be formed in the catheter shaft, by employing multiple mandrels when heat shrinking the outer tubular member onto the exterior of the inner tubular member. In such catheters it is preferred to have the secured section extend along the entire length of the catheter so that the adapter on the proximal end would be connected to the all of the individual lumens. In this manner the extra lumens may be employed to deliver drugs or other therapeutic fluids or be used as an additional inflation lumen.

The various components of the catheters and guidewires of the invention can he formed from a wide variety of conventional materials. The inner and outer plastic tubular members may be made from polyethylene, polyimide, polyvinyl chloride and other suitable plastic materials. The hypotubing may be formed of stainless steel, NiTi superelastic alloys or other suitable materials. Composite materials such as described in copending application Serial No. 07/241,047, filed September 6, 1988 (which is incorporated herein by reference thereto) may also be used. The balloon may be made from polyethylene, polyethylene terephthalate, olefinic ionomers such as Surlyn® sold by E.I. DuPont, deNemours & Co. and other polymers and other materials.

The dimensions of the catheters generally follow the dimensions of conventional intravascular catheters. For coronary use the length is typically about 135 cm and the maximum outer diameter of the outer tubular member is about 0.02 to about 0.06 inch (0.51-1.52 mm). In a presently preferred embodiment, the distal secured section of the outer tubular member is long enough (*e*.*g*. preferably about 10 to about 40 cm) to ensure that it is the only portion of the catheter body proximal to the balloon which exits the guiding catheter and enters the patient's coronary anatomy during intravascular procedures. In other embodiments, the secured section may extend along essentially the entire length of the catheter shaft. The transverse dimensions of the catheter may be larger with catheters for use in peripheral arteries and other locations.

## Claims

1. An elongated catheter for performing an angioplasty procedure comprising:
(a) an elongated catheter shaft (10) which has a distal shaft section with
an inner tubular member (11) having a distal end, a port in the distal end and a guidewire receiving lumen (12) extending in said inner tubular member (11) to said distal port;
an outer tubular member (13) having a portion of its inner periphery along a length (18) of the distal shaft section secured to the exterior of the inner tubular member (11); and
(b) a dilatation balloon (16) on the distal shaft section distal to said length and having an interior chamber therein:
**characterised in that**
the outer tubular member (13) is disposed about the inner tubular member (11) and the inner periphery of the outer tubular member (13) has a plurality of unsecured portions which are not secured to the exterior of the inner tubular member (11) resulting in a plurality of inner lumens (80) longitudinally extending along and between said inner and outer tubular members (11,13).

2. The elongated catheter of claim 1 wherein at least one of said inner lumens (80) is an inflation lumen in fluid communication with the interior chamber of said dilatation balloon (16).

3. The elongated catheter of claim 1 wherein the plurality of inner lumens (80) are parallel to and off-set from the guidewire lumen (12) defined by the inner tubular member (11).

4. The elongated catheter of claim 1 wherein the distal shaft section has along the length thereof one transverse dimension in a first direction substantially larger than a second transverse dimension in a second direction perpendicular to the first direction.

5. The elongated catheter of claim 4 wherein the dimension in the first transverse direction is at least 15% greater than the dimension in the second transverse direction perpendicular to.the first direction.

6. The elongated catheter of claim 1 wherein the guidewire receiving lumen (12) and the inner lumens (80) are in a stacked configuration within the distal shaft section.

7. The elongated catheter of claim 1 wherein the catheter shaft has a second port (42) which is located closer to the dilatation balloon (16) than the proximal end of the catheter shaft and which is in communication with the guidewire receiving lumen (12).

8. The elongated catheter of claim 1 wherein at least two of the plurality of inner lumens (80) extend to the proximal end of the catheter.

9. The elongated catheter of claim 1 wherein from 5% to 90% of the inner periphery of the outer tubular member (13) is secured to the exterior of the inner tubular member (11).

10. The elongated catheter of claim 9 wherein from 30% to 80% of the inner periphery of the outer tubular member (13) is secured to the exterior of the inner tubular member (11).

## Patentansprüche

1. Länglicher Katheter zur Durchführung eines angioplastischen Verfahrens, aufweisend:
(a) Einen länglichen Katheterschaft (10), der einen distalen Schaftabschnitt hat, mit
einem inneren rohrförmigen Element (11), das ein distales Ende, eine Öffnung in dem distalen Ende und
ein Lumen (12) zur Aufnahme eines Führungsdrahts hat, das sich in dem inneren rohrförmigen Element (11) zu dessen distalen Öffnung erstreckt;
einem äußeren rohrförmigen Element (13), das einen Abschnitt seines inneren Umfangs entlang einer Länge (18) des distalen Schaftabschnitts an dem Äußeren des inneren rohrförmigen Elements (11) befestigt hat; und
(b) einen Dilatationsbalon (16) an dem distalen Schaftabschnitt, distal zu der Länge und mit einer darin befindlichen, inneren Kammer:
**dadurch gekennzeichnet, daß**
das äußere rohrförmige Element (13) um das innere rohrförmige Element (11) angeordnet ist und der innere Umfang des äußeren rohrförmigen Elements (13) mehrere unbefestigte Abschnitte hat, die nicht an dem Äußeren des inneren rohrförmigen Elements (11) befestigt sind, was zu mehreren inneren Lumen (80) führt, die entlang und zwischen dem inneren und äußeren rohrförmigen Element (11, 13) verlaufen.

2. Länglicher Katheter nach Anspruch 1, bei welchem wenigstens eines der inneren Lumen (80) ein aufblähbares Lumen ist, das in fluidmäßiger Kommunikation mit der inneren Kammer des Dilatationsbalons (16) steht.

3. Länglicher Katheter nach Anspruch 1, bei welchem die mehreren inneren Lumen (80) parallel und versetzt sind zu dem Lumen (12) für einen Führungsdraht, das durch das innere rohrförmige Element (11) begrenzt ist.

4. Länglicher Katheter nach Anspruch 1, bei welchem der distale Schaftabschnitt entlang seiner Länge eine Querabmessung in einer Richtung hat, die im wesentlichen größer ist als eine zweite Querabmessung in einer zweiten Richtung, senkrecht zu der ersten Richtung.

5. Länglicher Katheter nach Anspruch 4, bei welchem die Abmessung in der ersten Querrichtung wenigstens 15 % größer ist als die Abmessung in der zweiten Querrichtung, senkrecht zu der ersten Richtung.

6. Länglicher Katheter nach Anspruch 1, bei welchem das Lumen (12) zur Aufnahme eines Führungsdrahts und die inneren Lumen (80) innerhalb des distalen Schaftabschnitts in einer gestapelten Ausgestaltung sind.

7. Länglicher Katheter nach Anspruch 1, bei welchem der Katheterschaft eine zweite Öffnung (42) hat, die näher zu dem Dilatationsbalon (16) angeordnet ist als das proximale Ende des Katheterschaftes und die in Kommunikation steht mit dem Lumen (12) zur Aufnahme eines Führungsdrahts.

8. Länglicher Katheter nach Anspruch 1, bei welchem wenigstens zwei der mehreren inneren Lumen (80) sich zu dem proximalen Ende des Katheters erstrecken.

9. Länglicher Katheter nach Anspruch 1, bei welchem von zwischen 5 % bis 90 % des inneren Umfangs des äußeren rohrförmigen Elements (13) an dem Äußeren des inneren rohrförmigen Elements (11) befestigt ist.

10. Länglicher Katheter nach Anspruch 9, bei welchem von zwischen 30 % bis 80 % des inneren Umfangs des äußeren rohrförmigen Elements (13) an dem Äußeren des inneren rohrförmigen Elements (11) befestigt ist.

## Revendications

1. Cathéter allongé pour l'exécution d'une procédure d'angioplastie, comportant :
(a) une tige allongée (10) de cathéter qui comporte un tronçon de tige distal avec
un élément tubulaire intérieur (11) ayant une extrémité distale, un orifice dans l'extrémité distale et une lumière (12) de réception d'un fil de guidage s'étendant dans ledit élément tubulaire intérieur (11) jusqu'audit orifice distal ;
un élément tubulaire extérieur (13) ayant une partie de sa périphérie intérieure, sur une longueur (18) du tronçon de tige distal, fixée à l'extérieur de l'élément tubulaire intérieur (11) ; et
(b) un ballonnet (16) à dilatation sur le tronçon de tige distal, situé du côté distal par rapport à ladite longueur et renfermant une chambre intérieure ;
**caractérisé en ce que**
l'élément tubulaire extérieur (13) est disposé autour de l'élément tubulaire intérieur (11) et la périphérie intérieure de l'élément tubulaire extérieur (13) comporte plusieurs parties non fixées qui ne sont pas fixées à l'extérieur de l'élément tubulaire intérieur (11), ce qui aboutit à la formation de plusieurs lumières intérieures (80) s'étendant longitudinalement le long des, et entre lesdits éléments tubulaires intérieur et extérieur (11, 13).

2. Cathéter allongé selon la revendication 1, dans lequel au moins l'une desdites lumières intérieures (80) est une lumière de gonflage en communication de fluide avec la chambre intérieure dudit ballonnet (16) à dilatation.

3. Cathéter allongé selon la revendication 1, dans lequel les multiples lumières intérieures (80) sont parallèles à, et décalées de, la lumière (12) pour le fil de guidage définie par l'élément tubulaire intérieur (11).

4. Cathéter allongé selon la revendication 1, dans lequel le tronçon de tige distal présente, sur sa longueur, une dimension transversale dans une première direction sensiblement plus grande qu'une seconde dimension transversale dans une seconde direction perpendiculaire à la première direction.

5. Cathéter allongé selon la revendication 4, dans lequel la dimension dans la première direction transversale est plus grande d'au moins 15% que la dimension dans la seconde direction transversale perpendiculaire à la première direction.

6. Cathéter allongé selon la revendication 1, dans lequel la lumière (12) de réception du fil de guidage et les lumières intérieures (80) sont dans une configuration superposée à l'intérieur du tronçon de tige distal.

7. Cathéter allongé selon la revendication 1, dans lequel la tige du cathéter présente un second orifice (42) qui est plus proche du ballonnet (16) à dilatation que l'extrémité proximale de la tige du cathéter et qui est en communication avec la lumière (12) de réception du fil de guidage.

8. Cathéter allongé selon la revendication 1, dans lequel au moins deux desdites multiples lumières intérieures (80) s'étendent jusqu'à l'extrémité proximale du cathéter.

9. Cathéter allongé selon la revendication 1, dans lequel 5% à 90% de la périphérie intérieure de l'élément tubulaire extérieur (13) sont fixés à l'extérieur de l'élément tubulaire intérieur (11).

10. Cathéter allongé selon la revendication 9, dans lequel 30% à 80% de la périphérie intérieure de l'élément tubulaire extérieur (13) sont fixés à l'extérieur de l'élément tubulaire intérieur (11).
